# EUROPEAN PATENT APPLICATION

(11) **EP 3 090 637 A1**
(43) Date of publication of application: **09.11.2016**
(21) Application number: 14875996.2
(22) Date of filing: 11.07.2014
(51) Int. Cl.: A23L 29/262, A23L 5/00

(54) **ENTERIC COATING COMPOSITION, ENTERIC COATING FILM AND FOOD PREPARATION**

(30) Priority: 31.12.2013 KR 20130169403
(71) Applicant: LOTTE Fine Chemical Co., Ltd., Ulsan, 44714 (KR)
(72) Inventor: CHA, Jae Uk, Seoul 152-090 (KR); LEE, Sung Wan, Incheon 402-803 (KR); HONG, Jun Kee, Yongin-si Gyeonggi-do 448-728 (KR); CHA, Ja Hyun, Incheon 405-734 (KR); KO, Won Hwa, Incheon 403-862 (KR)
(74) Representative: Kador & Partner
(86) International application number: PCT/KR2014/006254
(87) International publication number: WO 2015/102189

(57) **Abstract**

Disclosed are an enteric coating composition, an enteric coating film, and a food formulation. The disclosed enteric coating composition comprises an enteric cellulose-based compound, a pH controlling agent, a plasticizer for food, and a solvent, wherein the plasticizer for food comprises acetylated monoglyceride, triacetin, or a mixture thereof, and a content of the pH controlling agent and a content of the plasticizer for food ranges respectively from 10 parts to 40 parts by weight and from 5 parts to 25 parts by weight on the basis of 100 parts by weight of the enteric cellulose-based compound.

## Description

### 1. TECHNICAL FIELD

The inventive concept relates to an enteric coating composition, an enteric coating film, and a food formulation, and more particularly, to an enteric coating composition that may be used for coating a food formulation, an enteric coating film, and a food formulation.

### BACKGROUND ART

Cellulose-based compounds such as hydroxypropyl methylcellulose phthalate (HPMCP) or hydroxypropyl methylcellulose acetate succinate (HPMCAS) are used in preparation of an enteric coating film. Conventionally, an enteric coating film has been prepared by dissolving the cellulose-based compound in an organic solvent or a mixed solvent including an organic solvent to prepare an enteric coating composition, and then coating the enteric coating composition on a food formulation. However, in this case, the residual organic solvent from the preparation process caused environmental problems and safety issues.

In order to resolve the environmental problems or and safety issues, methods of preparing an enteric coating film by dissolving the cellulose-based compound with an alkalifying agent such as an aqueous sodium hydroxide solution or ammonia water to prepare an enteric coating composition, and then coating the enteric coating composition on a food formulation have been tried. However, in this case, some of the alkalifying agent remained on the enteric coating film thus prepared, and when the enteric coating film was formed thin, coating quality deteriorated, and thus the enteric coating film was not available to be used.

Also, the enteric coating composition including a general cellulose-based compound has insufficient plasticity, which results in an uneven surface of an enteric coating film prepared by using the composition, and when the enteric coating film is stored for a long time period, the coating film may crack. To resolve the problems, a pharmaceutical plasticizer is added to the enteric coating composition. However, when the pharmaceutical plasticizer is added to the enteric coating composition, the enteric coating composition may not be used in a food formulation.

### DETAILED DESCRIPTION OF THE INVENTIVE CONCEPT

### TECHNICAL PROBLEM

The inventive concept provides an enteric coating composition that may be used for coating a food formulation.

The inventive concept provides an enteric coating film prepared by using the enteric coating composition.

The inventive concept provides a food formulation including the enteric coating film.

### TECHNICAL SOLUTION

According to an aspect of the inventive concept, there is provided an enteric coating composition including an enteric cellulose-based compound; a pH controlling agent; a plasticizer for food; and a solvent, wherein the plasticizer for food comprises acetylated monoglyceride, triacetin, or a mixture thereof, and a content of the pH controlling agent and a content of the plasticizer for food range respectively from 10 parts to 40 parts by weight and from 5 parts to 25 parts by weight on the basis of 100 parts by weight of the enteric cellulose-based compound.

The enteric cellulose-based compound may include hydroxypropyl methylcellulose phthalate (HPMCP), hydroxypropyl methylcellulose acetate succinate (HPMCAS), cellulose acetate phthalate (CAP), a derivative thereof, or a mixture thereof.

The pH controlling agent may be ammonium bicarbonate (AHC).

The solvent may be 100% water, and a content of the solvent ranges from 100 part to 2,000 parts by weight based on 100 parts by weight of the total weight of the enteric cellulose-based compound, the pH controlling agent, and the plasticizer for food.

According to another aspect of the inventive concept, there is provided an enteric coating film including an enteric cellulose-based compound; and a plasticizer for food, wherein a content of the plasticizer for food ranges from 5 parts to 25 parts by weight based on 100 parts by weight of the enteric cellulose-based compound.

According to another aspect of the inventive concept, there is provided a food formulation including the enteric coating film.

### ADVANTAGEOUS EFFECTS OF THE INVENTION

According to an embodiment of the inventive concept, provided is an enteric coating composition having a fast dissolving rate and a homogeneous composition.

According to another embodiment of the inventive concept, provided is an enteric coating film that does not have residual foreign materials or impurities on a surface thereof, does not have salting out, or does not cause odor and color problems.

According to another embodiment of the inventive concept, provided is a food formulation that has a high coating quality and excellent long-term storage stability by including a plasticizer for food.

### MODE OF THE INVENTIVE CONCEPT

Hereinafter, an enteric coating composition according to an embodiment of the inventive concept will be described.

The enteric coating composition includes an enteric cellulose-based compound, a pH controlling agent, a plasticizer for food, and a solvent.

As used herein, the term "enteric" refers to a property that does not disintegrate and dissolute in the gastric juice condition (at pH around 1.2) for 2 hours but disintegrates and dissolutes in the small intestinal juice condition (at pH around 6.8) within a short time period of 1 hour or less.

The enteric cellulose-based compound may include hydroxypropyl methylcellulose phthalate (HPMCP), hydroxypropyl methylcellulose acetate succinate (HPMCAS), cellulose acetate phthalate (CAP), a derivative thereof, or a mixture thereof.

The pH controlling agent assists the enteric cellulose-based compound to be dissolved in the solvent.

The pH controlling agent may be ammonium bicarbonate (AHC).

A content of the pH controlling agent ranges from 10 parts to 40 parts by weight based on 100 parts by weight of the enteric cellulose-based compound. When the content of the pH controlling agent is lower than 10 parts by weight based on 100 parts by weight of the enteric cellulose-based compound, the enteric coating composition may include undissolved components, which may not allow a homogenous composition. Also, when the content of the pH controlling agent is greater than 40 parts by weight based on 100 parts by weight of the enteric cellulose-based compound, the pH controlling agent may not be completely removed after coating with the enteric coating composition and may remain in the enteric coating film, which may cause salting out.

The plasticizer for food levels a surface of an enteric coating film prepared by using the enteric coating composition and prevents the enteric coating film from cracking even when the enteric coating film is stored for a long time period. Also, use of the plasticizer for food is approved by European food safety authority (EFSA), and thus using the plasticizer for food for forming a coating film of a food formulation is not subject to legal restrictions.

The plasticizer for food includes acetylated monoglyceride, triacetin, or a mixture thereof.

A content of the plasticizer for food ranges from 5 parts to 25 parts by weight based on 100 parts by weight of the enteric cellulose-based compound. When the content of the plasticizer for food is less than 5 parts by weight based on 100 parts by weight of the enteric cellulose-based compound, an enteric film having evenly flat surface and excellent long-term storage stability may be difficult to be obtained. Also, when the content of the plasticizer for food is greater than 25 parts by weight based on 100 parts by weight of the enteric cellulose-based compound, a coating film prepared by using a coating composition that includes the plasticizer for food disintegrates even at pH of 1.2 due to a water soluble property of the plasticizer for food, which may not allow the coating film to have an enteric function.

The solvent may be 100% water. However, the solvent may include impurities unavoidably contained in general water.

A content of the solvent may range from 100 parts to 2,000 parts by weight based on 100 parts by weight of the total weight of the enteric cellulose-based compound, the pH controlling agent, and the plasticizer for food. When the content of the solvent is within this range, components constituting the enteric coating composition may homogenously mixed, and thus an enteric coating film of high quality may be obtained.

The enteric coating composition may further include an additive such as a lubricant, a colorant, a sunscreen agent, a solubilizing agent, a gelling agent, or a mixture thereof.

All components of the enteric coating composition (e.g., the enteric cellulose-based compound, the pH controlling agent, the plasticizer for food, and the solvent) may be homogeneously mixed so that the enteric coating composition may have a homogenous composition. That is, the enteric coating composition may be a solution.

The enteric coating composition may be prepared by the following method.

In one embodiment, the enteric coating composition may be prepared by dissolving the pH controlling agent in the solvent to obtain a first solution, dissolving the enteric cellulose-based compound in the first solution to obtain a second solution, and dissolving the plasticizer for food in the second solution.

In another embodiment, the enteric coating composition may be prepared by adding the enteric cellulose-based compound, the pH controlling agent, and the plasticizer for food to the solvent altogether at the same time and dissolving them in the solvent at the same time.

In another embodiment, the enteric coating composition may be prepare by separately dissolving each of the enteric cellulose-based compound, the pH controlling agent, and the plasticizer for food in the solvent to respectively prepare a solution of the enteric cellulose-based compound, a solution of the pH controlling agent, and a solution of plasticizer for food; and then mixing the solutions.

Hereinafter, the enteric coating film according to an embodiment of the inventive concept will be described.

The enteric coating film includes the enteric cellulose-based compound and the plasticizer for food. However, the enteric coating film does not include the pH controlling agent. Here, the reason why the enteric coating film does not include the pH controlling agent is because the pH controlling agent will be decomposed into carbon dioxide and ammonia gas and be removed during a drying process after coating the enteric coating composition on a food formulation.

A content of the plasticizer for food in the enteric coating film ranges from 5 parts to 25 parts by weight based on 100 parts by weight of the enteric cellulose-based compound.

Although the enteric coating film is prepared by using safe food materials only, the enteric coating film has a pH-dependent solubility and thus may have the enteric property equivalent to that of an enteric coating film formed of a pharmaceutical compound.

The enteric coating film may be prepared as follows.

For example, the enteric coating film may be prepared by coating the enteric coating composition on a food formulation, such as a tablet or a capsule, and then drying the resultant.

The drying process of the enteric coating composition may be performed by, for example, reduced-pressure evaporation, spray drying, or air or natural drying.

The drying process may be performed at a temperature in a range of 15°C to 150°C, or, for example, 50°C to 60°C. When the drying process is performed at a temperature within this range, the enteric coating film is not degenerated, and the solvent may be easily removed during formation of the enteric coating film.

The enteric coating film thus obtained may be additionally washed to remove impurities therefrom.

Hereinafter, the food formulation according to an embodiment of the inventive concept will be described.

The food formulation includes the enteric coating film.

The food formulation may be a tablet or a capsule.

The capsule may be a hard capsule or a soft capsule.

The food formulation may be a health functional food formulation.

Thereinafter, one or more embodiments of the inventive concept will be described in detail with reference to the following examples. However, these examples are not intended to limit the scope of the inventive concept.

### Examples 1 to 9 and Comparative Examples 1 to 13: Preparation of coating composition and coating film

### (Preparation of coating composition)

### Ammonium bicarbonate (AHC) was dissolved in water to obtain a first solution. Then, an enteric cellulose-based compound was added to the first solution, and the resultant was stirred until the enteric cellulose-based compound was completely dissolved. As a result, a second solution was obtained. Subsequently, a plasticizer was added to the second solution. As a result, a coating composition was obtained. A type and a content of the enteric cellulose-based compounds used in each of Examples and Comparative Examples and use, a type, and a content of the plasticizer are shown in Table 1.

**[Table 1]**

| | Water (part by weight) | AHC (part by weight) | Enteric cellulose-based compound | | Plasticizer | | |
|---|---|---|---|---|---|---|---|
| | | | Type | Content (part by weight) | Use | Type | Content (part by weight) |
| Example 1 | 1000 | 20 | HPMCP^{*1} | 100 | for food | AMG^{*3} | 5 |
| Example 2 | 1000 | 20 | HPMCP | 100 | for food | AMG | 10 |
| Example 3 | 1000 | 20 | HPMCP | 100 | for food | AMG | 15 |
| Example 4 | 1000 | 20 | HPMCP | 100 | for food | AMG | 25 |
| Example 5 | 1000 | 20 | HPMCP | 100 | for food | TA^{*4} | 5 |
| Example 6 | 1000 | 20 | HPMCP | 100 | for food | TA | 10 |
| Example 7 | 1000 | 20 | HPMCP | 100 | for food | TA | 15 |
| Example 8 | 1000 | 20 | HPMCP | 100 | for food | TA | 25 |
| Example 9 | 1000 | 20 | HPMCAS^{*2} | 100 | for food | TA | 15 |
| Comparative Example 1 | 1000 | 20 | HPMCP | 100 | for pharmaceuticals | TEC^{*5} | 5 |
| Comparative Example 2 | 1000 | 20 | HPMCP | 100 | for pharmaceuticals | TEC | 10 |
| Comparative Example 3 | 1000 | 20 | HPMCP | 100 | for pharmaceuticals | TEC | 15 |
| Comparative Example 4 | 1000 | 20 | HPMCP | 100 | for pharmaceuticals | TEC | 25 |
| Comparative Example 5 | 1000 | 20 | HPMCP | 100 | for food | PG^{*6} | 5 |
| Comparative Example 6 | 1000 | 20 | HPMCP | 100 | for food | PG | 10 |
| Comparative Example 7 | 1000 | 20 | HPMCP | 100 | for food | PG | 15 |
| Comparative Example 8 | 1000 | 20 | HPMCP | 100 | for food | PG | 25 |
| Comparative Example 9 | 1000 | 20 | HPMCP | 100 | for food | G^{*7} | 5 |
| Comparative Example 10 | 1000 | 20 | HPMCP | 100 | for food | G | 10 |
| Comparative Example 11 | 1000 | 20 | HPMCP | 100 | for food | G | 15 |
| Comparative Example 12 | 1000 | 20 | HPMCP | 100 | for food | G | 25 |
| Comparative Example 13 | 1000 | 20 | HPMCP | 100 | - | - | 0 |

| | | | | | | | |
|---|---|---|---|---|---|---|---|
| *1: HPMCP (HPMCP HP-55, available from Samsung Fine Chemicals Co., Ltd.) *2: HPMCAS (HPMCAS AS-LF, available from Shin-Etsu) *3: Acetylated monoglyceride (Myvacet, available from Kerry) *4: Triacetin (available from Junsei) *5: Triethyl citrate (available from Merck) *6: Propylene glycol (available from Samchun) *7: Glycerol (available from Junsei) | | | | | | | |

### (Preparation of coating film)

The coating composition was connected to a coater (Hi-coater, available from Freund) and was coated on each of an Aspirin tablet (Aspirin Tab 100 mg, with engraved logo thereon, available from Bayer) and a soft gelatin capsule (Well-being Omega3 1000 mg, a soft capsule, available from Chong Kun Dang Healthcare) under conditions including a exhalation temperature of 53°C, an inhalation temperature of 40°C, a feed rate of the coating composition of 3 g/min, an in-air flow rate of 0.8 m³/min, a pan static of -20 Pa, a pan speed of 25 rpm, and a spray air pressure of 0.2 MPa. As a result, a coating film was obtained. When the coating composition was coated on the Aspirin tablet, the coating was performed until 5 wt% of a coating amount of the coating composition was obtained based on 100 wt% of the Aspirin tablet before the coating process. Also, when the coating composition was coated on the soft gelatin capsule, the coating was performed until 10 wt% of a coating amount of the coating composition was obtained based on 100 wt% of the soft gelatin capsule before the coating process.

### Evaluation Example

Physical properties of the coating compositions and coating films prepared in Examples 1 to 9 and Comparative Examples 1 to 13 were each evaluated as follows, and the results are shown in Table 2.

### Evaluation Example 1: Coating characteristics (logo bridge goodness and coating film transparency) evaluation

### (Evaluation of logo bridge goodness)

States of the coating films formed on the engraved logos of the coated Aspirin tablets were each observed with naked eyes to evaluate logo bridge goodness according to a 5-point scaling method. Specifically, the logo bridge goodness was evaluated depending on how deeply the coating film coated the inside of the logo in a manner that a state in which the coating film was completely adhered on the whole inner surface of the logo was evaluated as 5 points; and a state in which the coating film was not adhered on the inner surface of the logo at all or above the inner surface was evaluated as 1 point.

### (Evaluation of coating film transparency)

Transparencies of the coated soft gelatin capsules were each observed with naked eyes to evaluate coating film transparency according to a 5-point scaling method. Specifically, the coating film transparency was evaluated in a manner that, when the coated soft gelatin capsule was completely transparent, a transparency of the coated soft gelatin capsule was evaluated as 5 points; and, when the coated soft gelatin capsule was completely opaque, a transparency of the coated soft gelatin capsule was evaluated as 1 point.

### Evaluation Example 2: Evaluation of convenience in process

In the process of preparing the coating compositions according to Examples 1 to 9 and Comparative Examples 1 to 13, a dissolving rate of each of the enteric cellulose-based compounds was measured, and thus convenience in the coating process was evaluated as follows.
○: The enteric cellulose-based compound is completely dissolved into a transparent state within 2 hours from the entering point.
X: The enteric cellulose-based compound is not completely dissolved and remains after 2 hours from the entering point.

### Evaluation Example 3: Disintegration evaluation in gastric juice (at pH of 1.2) and in artificial intestinal juice (at pH of 6.8)

Disintegration characteristics of the coated Aspirin tables and the coated soft gelatin capsules prepared in Examples 1 to 9 and Comparative Examples 1 to 13 were each comparatively evaluated as follows. That is, an elution test was performed according to a disintegration test method of the Korean Pharmacopeia (9^{th} edition) at a temperature of 37°C. 900 ml of each of artificial gastric juice (at pH of 1.2, a hydrochloride buffer solution) and artificial intestinal juice (at pH of 6.8, a 50 mM phosphate buffer solution) was used as an effluent. The disintegration test started in the artificial gastric juice at pH of 1.2, after 2 hours, the artificial gastric juice was replaced with the artificial intestinal juice at pH of 6.8, and the disintegration test continued for another 1 hour thereafter. Samples that did not disintegrated in the artificial gastric juice (at pH of 1.2) for 2 hours but disintegrated in the artificial intestinal juice (at pH of 6.8) within 1 hour were evaluated as samples having enteric property.
○: having enteric property
X: having no enteric property

### Evaluation Example 4: Evaluation of long term storage stability

25 capsules of each of the coated soft gelatin capsules prepared in Examples 1 to 9 and Comparative Examples 1 to 13 were stored under room temperature condition and acceleration condition for 60 days. The number of the soft gelatin capsules having a burst coating film was shown by percent to evaluate the long term storage stability. As used herein, the term "room temperature condition" denotes that 25 capsules of each of the soft gelatin capsules are contained in a sealed vessel and stored in a thermo-hygrostat at a temperature of 25°C and a relative humidity of 50%, and the term "acceleration condition" denotes that 25 capsules of each of the soft gelatin capsules are contained in a sealed vessel and stored in a thermo-hygrostat at a temperature of 40°C and a relative humidity of 75%.

**[Table 2]**

| | Logo bridge goodness | Transparency of coating film | Convenience in process | Enteric property | Long-term storage stability (%) |
|---|---|---|---|---|---|
| Example 1 | 3 | 2 | ○ | ○ | 96 |
| Example 2 | 3 | 2 | ○ | ○ | 92 |
| Example 3 | 3 | 2 | ○ | ○ | 100 |
| Example 4 | 3 | 2 | ○ | ○ | 100 |
| Example 5 | 5 | 5 | ○ | ○ | 92 |
| Example 6 | 5 | 5 | ○ | ○ | 96 |
| Example 7 | 5 | 5 | ○ | ○ | 100 |
| Example 8 | 5 | 5 | ○ | ○ | 100 |
| Example 9 | 5 | 2 | ○ | ○ | 96 |
| Comparative Example 1 | 5 | 4 | ○ | ○ | 92 |
| Comparative Example 2 | 5 | 4 | ○ | ○ | 96 |
| Comparative Example 3 | 5 | 5 | ○ | ○ | 100 |
| Comparative Example 4 | 5 | 5 | ○ | ○ | 100 |
| Comparative Example 5 | 2 | 2 | ○ | ○ | 92 |
| Comparative Example 6 | 2 | 2 | ○ | ○ | 92 |
| Comparative Example 7 | 2 | 2 | ○ | ○ | 92 |
| Comparative Example 8 | 2 | 2 | ○ | ○ | 96 |
| Comparative Example 9 | 1 | 1 | ○ | ○ | 88 |
| Comparative Example 10 | 1 | 1 | ○ | ○ | 80 |
| Comparative Example 11 | 1 | 1 | ○ | ○ | 88 |
| Comparative Example 12 | 1 | 1 | ○ | ○ | 92 |
| Comparative Example 13 | 1 | 1 | ○ | ○ | 72 |

Referring to Table 2, all physical properties of the coated soft gelatin capsules prepared in Examples 1 to 9 by using a plasticizer for food were equivalent to those of the coated soft gelatin capsules prepared in Comparative Examples 1 to 4 by using a plasticizer for pharmaceutics. Also, each of the coated soft gelatin capsules prepared in Examples 1 to 9 by using a plasticizer for food includes a plasticizer for food and thus may be used in a food formulation. However, each of the coated soft gelatin capsules prepared in Comparative Examples 1 to 4 by using a plasticizer for pharmaceutics includes a plasticizer for pharmaceutics, not a plasticizer for food, and thus may not be used in a food formulation. Also, each of the coated soft gelatin capsules prepared in Examples 1 to 9 had at least one physical property among logo bridge goodness, coating film transparency, and long term storage stability better than that of each of the coated soft gelatin capsules prepared in Comparative Examples 5 to 13.

While the inventive concept has been particularly shown and described with reference to exemplary embodiments thereof, it will be understood that various changes in form and details may be made therein without departing from the spirit and scope of the following claims.

## Claims

1. An enteric coating composition comprising:
an enteric cellulose-based compound;
a pH controlling agent;
a plasticizer for food; and
a solvent,
wherein the plasticizer for food comprises acetylated monoglyceride, triacetin, or a mixture thereof, and
a content of the pH controlling agent and a content of the plasticizer for food range respectively from 10 parts to 40 parts by weight and from 5 parts to 25 parts by weight on the basis of 100 parts by weight of the enteric cellulose-based compound.

2. The enteric coating composition of claim 1, wherein the enteric cellulose-based compound comprises hydroxypropyl methylcellulose phthalate (HPMCP), hydroxypropyl methylcellulose acetate succinate (HPMCAS), cellulose acetate phthalate (CAP), a derivative thereof, or a mixture thereof.

3. The enteric coating composition of claim 1, wherein the pH controlling agent is ammonium bicarbonate (AHC).

4. The enteric coating composition of claim 1, wherein the solvent is 100% water, and a content of the solvent ranges from 100 part to 2,000 parts by weight based on 100 parts by weight of the total weight of the enteric cellulose-based compound, the pH controlling agent, and the plasticizer for food.

5. An enteric coating film comprising:
an enteric cellulose-based compound; and
a plasticizer for food,
wherein a content of the plasticizer for food ranges from 5 parts to 25 parts by weight based on 100 parts by weight of the enteric cellulose-based compound.

6. A food formulation comprising the enteric coating film of claim 5.
